# EUROPEAN PATENT APPLICATION

(11) **EP 3 666 318 A1**
(43) Date of publication of application: **17.06.2020**
(21) Application number: 18214328.9
(22) Date of filing: 20.12.2018
(51) Int. Cl.: A61M 15/06, A24F 1/00, A61M 11/04

(54) **VAPORIZER HAVING ONE-WAY AIRFLOW VALVE AND ELECTRONIC CIGARETTES HAVING THE SAME**

(30) Priority: 11.12.2018 US 201816215979
(71) Applicant: Shenzhen Kanger Technology Co., Ltd., 518103 Shenzhen (CN)
(72) Inventor: ZHU, Xiaochun, SHENZHEN, GUANGDONG 518103 (CN)
(74) Representative: Wang, Bo

(57) **Abstract**

Present disclosure relates to a one-way airflow valve and electronic cigarettes having the one-way airflow valve. In certain embodiments, the electronic cigarette includes: a mouthpiece assembly, an E-liquid storage tank for storing E-liquid, a vaporizer assembly having a one-way airflow valve, an electric power connector for receiving electric power, and a condensed E-liquid vapor collection slot for collecting condensed E-liquid vapor to be reused. The mouthpiece assembly and vaporizer assembly form an airflow path. When user connects electric power source to the electric power connector, and sucks electronic cigarette through a mouthpiece, air pressure inside electronic cigarette is reduced which creates air pressure difference, such air pressure difference pushes a one-way airflow blocker upwards to open airflow path, and when user stops sucking, one-way airflow blocker returns to a first position under resilient force of the one-way airflow valve spring to close the airflow path.

## Description

### FIELD

The present disclosure generally relates to the field of electronic cigarettes, and more particularly to a one-way airflow valve, electronic cigarettes having the one-way airflow valve, and methods of using the electronic cigarettes having the one-way airflow valve.

### BACKGROUND

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

It is well known that smoking cigarette is harmful to smoker's health. The active ingredient in a cigarette is mainly nicotine. During smoking, nicotine, along with tar aerosol droplets produced in the cigarette burning, are breathed into the alveolus and absorbed quickly by the smoker. Once nicotine is absorbed into the blood of the smoker, nicotine then produces its effect on the receptors of the smoker's central nervous system, causing the smoker relax and enjoy an inebriety similar to that produced by an exhilarant.

Leaking condensed E-liquid vapor out of vaporizer is a common problem for conventional electronic cigarettes. When E-liquid vapor is not fully consumed by a user, such E-liquid vapor may be liquified due to condensation and may be accumulated inside vaporizer. The excessive accumulation of this liquid causes leaking. Although there are vapor amount adjustment mechanisms in certain electronic cigarette, but these vapor amount adjustment mechanisms are usually manual and mechanical, it does not allow the electronic cigarette users to adjust the vapor amount automatically. It is desirable that the electronic cigarette has an ability to reduce and eliminate the condensation of E-liquid vapor and allow user to adjust the vapor amount automatically.

Therefore, an unaddressed need exists in the art to address the aforementioned deficiencies and inadequacies.

### SUMMARY

In one aspect, the present disclosure relates to a one-way airflow valve. The one-way airflow valve is used in a vaporizer assembly of an electronic cigarette. In certain embodiments, the one-way airflow valve includes: a cylindrical one-way airflow valve body, a one-way airflow valve cap, a one-way airflow valve spring, and a one-way airflow blocker. The cylindrical one-way airflow valve body has a top end and a bottom end. The cylindrical one-way airflow valve body defines a set of air outlet openings for discharging airflow through the one-way airflow valve. The one-way airflow valve cap closes the top end of the cylindrical one-way airflow valve body. The bottom end forms a one-way airflow air-intake opening. When the one-way airflow valve is positioned in a vaporizer assembly of an electronic cigarette, the vaporizer assembly forms an airflow path from the one-way airflow air-intake opening at the bottom end of the one-way airflow valve through the set of air outlet openings. The one-way airflow blocker is used to open and close the airflow path. When the one-way airflow blocker is in a first position, the airflow path is closed, and when the one-way airflow blocker is in a second position, the airflow path is open. The one-way airflow valve spring keeps the one-way airflow blocker in the first position. When a user sucks the electronic cigarette through a mouthpiece, air pressure inside the electronic cigarette is reduced. The reduction of air pressure creates an air pressure difference and the air pressure difference pushes the one-way airflow blocker of the one-way airflow valve upwards to the second position and opens the airflow path. When the user stops sucking, the one-way airflow blocker returns to the first position under resilient force of the one-way airflow valve spring to close the airflow path.

In certain embodiments, the set of air outlet openings is evenly defined around a side wall of the cylindrical one-way airflow valve body. In one embodiment, the set of air outlet openings includes: a first air-outlet opening, a second air-outlet opening, a third air-outlet opening, and a fourth air-outlet opening.

In certain embodiments, the one-way airflow valve spring includes a first end attached to the one-way airflow valve cap, and a second end attached to the one-way airflow blocker. Resilient force of the one-way airflow valve spring keeps the one-way airflow blocker in the first position to close the airflow path. The resilient force of the one-way airflow valve spring is selected to facilitate opening and closing of the airflow path and allow the user to adjust the amount of airflow through the one-way airflow valve and amount of E-liquid vaper according to suction force applied in real-time automatically.

The one-way airflow blocker is made of: metal, plastic, silicon rubber or wood.

In another aspect, the present disclosure relates to an electronic cigarette. In certain embodiments, the electronic cigarette includes: a mouthpiece assembly for a user to enjoy E-liquid vapor, an E-liquid storage tank for storing E-liquid, a vaporizer assembly having a one-way airflow valve, an electric power connector for receiving electric power from an electric power source and to provide the electric power to the vaporizer assembly, and a condensed E-liquid vapor collection slot for collecting condensed E-liquid vapor to be reused. The mouthpiece assembly and the vaporizer assembly form an airflow path from a one-way airflow air-intake opening at a bottom end of the one-way airflow valve through a set of air outlet openings of the one-way airflow valve. When the user connects the electric power source to the electric power connector, and sucks the electronic cigarette through the mouthpiece assembly, air pressure inside the electronic cigarette is reduced, the reduction of air pressure creates an air pressure difference, and the air pressure difference pushes a one-way airflow blocker of the one-way airflow valve upwards to a second position and opens the airflow path, and when the user stops sucking, the one-way airflow blocker returns to a first position under resilient force of the one-way airflow valve spring to close the airflow path.

In certain embodiments, the mouthpiece assembly includes a mouthpiece positioned on a mouthpiece mounting base. The mouthpiece is sealed by a mouthpiece sealing ring.

In certain embodiments, the E-liquid storage tank includes: an E-liquid storage tank top outer cover, an E-liquid storage tank top inner cover, an E-liquid storage tank external wall, an E-liquid storage tank base, and a vaporizer assembly body.

In certain embodiments, the mouthpiece mounting base is slidably attached to the E-liquid storage tank top outer cover. The E-liquid storage tank top inner cover defines an E-liquid refill opening and a center vapor outlet opening. The E-liquid storage tank top inner cover includes a vapor outlet and E-liquid refill cover. The vapor outlet and E-liquid refill cover defines an E-liquid refill opening corresponding to the E-liquid refill opening of the E-liquid storage tank top inner cover and a center vapor outlet opening corresponding to the center vapor outlet opening of the E-liquid storage tank top inner cover. The E-liquid storage tank top outer cover and the E-liquid storage tank top inner cover form a top cover of the E-liquid storage tank. The E-liquid storage tank base forms a bottom cover of the E-liquid storage tank.

In certain embodiments, the E-liquid storage tank external wall has a top end, and a bottom end. The top end of the E-liquid storage tank external wall is sealed by an E-liquid storage tank top sealing ring, and the bottom end of the E-liquid storage tank external wall is sealed by an E-liquid storage tank bottom sealing ring.

In certain embodiments, the vaporizer assembly body is covered by a vaporizer assembly top vapor outlet on a top end of the vaporizer assembly body, and a one-way airflow valve mounting base at a bottom end of the vaporizer assembly body. The vaporizer assembly body and the vaporizer assembly top vapor outlet form an E-liquid storage tank internal wall. The vaporizer assembly body defines a set of vaporizer E-liquid openings to allow the E-liquid stored in the E-liquid storage tank to flow through the set of vaporizer E-liquid openings into the vaporizer assembly.

In certain embodiments, the E-liquid is filled through the E-liquid refill opening of the E-liquid storage tank top inner cover and the E-liquid refill opening of the vapor outlet and E-liquid refill cover, and the E-liquid flows to the vaporizer assembly through the set of vaporizer E-liquid openings.

In certain embodiments, the vaporizer assembly further includes: an outer cylindrical E-liquid medium, an inner cylindrical E-liquid medium, a heating element, and a vaporizer base. The vaporizer base has a cylindrical wall defining a set of vaporizer base E-liquid openings. The outer cylindrical E-liquid medium is positioned outside of the cylindrical wall of the vaporizer base, and the inner cylindrical E-liquid medium is positioned inside of the cylindrical wall of the vaporizer base. The inner cylindrical E-liquid medium receives the E-liquid from the E-liquid storage tank through the outer cylindrical E-liquid medium and the set of vaporizer E-liquid openings. The heating element heats the E-liquid in the inner cylindrical E-liquid medium to generate the E-liquid vapor.

In certain embodiments, the one-way airflow valve includes: a cylindrical one-way airflow valve body, a one-way airflow valve cap, a one-way airflow valve spring, and the one-way airflow blocker. The cylindrical one-way airflow valve body has a top end and a bottom end. The cylindrical one-way airflow valve body defines a set of air outlet openings for discharging airflow through the one-way airflow valve. The one-way airflow valve cap closes the top end of the cylindrical one-way airflow valve body. The bottom end forms the one-way airflow air-intake opening. When the one-way airflow valve is positioned in a vaporizer assembly of the electronic cigarette, the vaporizer assembly forms the airflow path from the one-way airflow air-intake opening at the bottom end of the one-way airflow valve through the set of air outlet openings. The one-way airflow blocker is used to open and close the airflow path. When the one-way airflow blocker is in a first position, the airflow path is closed, and when the one-way airflow blocker is in a second position, the airflow path is open. The one-way airflow valve spring keeps the one-way airflow blocker in the first position. When a user sucks the electronic cigarette through a mouthpiece, air pressure inside the electronic cigarette is reduced. The reduction of air pressure creates an air pressure difference, and the air pressure difference pushes the one-way airflow blocker of the one-way airflow valve upwards to the second position and opens the airflow path. When the user stops sucking, the one-way airflow blocker returns to the first position under resilient force of the one-way airflow valve spring to close the airflow path.

In certain embodiments, the set of air outlet openings is evenly defined around a side wall of the cylindrical one-way airflow valve body. In one embodiment, the set of air outlet openings includes: a first air-outlet opening, a second air-outlet opening, a third air-outlet opening, and a fourth air-outlet opening.

In certain embodiments, the one-way airflow valve spring includes a first end attached to the one-way airflow valve cap, and a second end attached to the one-way airflow blocker. Resilient force of the one-way airflow valve spring keeps the one-way airflow blocker in the first position to close the airflow path. The resilient force of the one-way airflow valve spring is selected to facilitate opening and closing of the airflow path and allow the user to adjust the amount of airflow through the one-way airflow valve and amount of E-liquid vaper according to suction force applied in real-time automatically The one-way airflow blocker is made of: metal, plastic, silicon rubber or wood.

In certain embodiments, the heating element is made of one or more resistive materials. When the heating element is powered by the electric power source through a positive electric power terminal and a negative electric power terminal of the electric power connector, the heating element heats the E-liquid siphoned through the outer cylindrical E-liquid medium and the inner cylindrical E-liquid medium to generate the E-liquid vapor.

In certain embodiments, the electric power connector includes: the positive electric power terminal, the negative electric power terminal, and an electric power insulator ring. The positive electric power terminal connects the heating element to a positive terminal of the electric power source. The negative electric power terminal connects the heating element to a negative terminal of the electric power source. The electric power insulator ring is positioned between the positive electric power terminal and the negative electric power terminal to insulate the positive electric power terminal and the negative electric power terminal. In certain embodiments, the vaporizer base forms the negative electric power terminal.

In yet another aspect, the present disclosure relates to a method of using an electronic cigarette having a one-way airflow valve. In certain embodiments, the method of using the electronic cigarette includes: sliding off, by a user, a mouthpiece mounting base of the electronic cigarette and filling E-liquid into an E-liquid storage tank through an E-liquid refill opening on an E-liquid storage tank top inner cover, and connecting, by the user, an electric power source to an electric power connector of the electronic cigarette. The method of using the electronic cigarette also includes: turning on, by the user, the electric power source to power a vaporizer assembly of the electronic cigarette having the one-way airflow valve, sucking, by the user, through a mouthpiece to open an airflow path through the one-way airflow valve and enjoying the vapor generated by the vaporizer assembly, and stopping sucking through the mouthpiece to close the airflow path through the one-way airflow valve such that the airflow through the vaporizer assembly stops.

In certain embodiments, the electronic cigarette includes: a mouthpiece assembly for a user to enjoy E-liquid vapor, the E-liquid storage tank for storing E-liquid, the vaporizer assembly having the one-way airflow valve, and the electric power connector for receiving electric power from an electric power source and to provide the electric power to the vaporizer assembly. The mouthpiece assembly and the vaporizer assembly form the airflow path from a one-way airflow air-intake opening at a bottom end of the one-way airflow valve through a set of air outlet openings of the one-way airflow valve. When the user connects the electric power source to the electric power connector, and sucks the electronic cigarette through the mouthpiece assembly, air pressure inside the electronic cigarette is reduced, the reduction of air pressure creates an air pressure difference, and the air pressure difference pushes a one-way airflow blocker upwards to a second position and opens the airflow path. When the user stops sucking, the one-way airflow blocker returns to a first position under resilient force of the one-way airflow valve spring to close the airflow path.

In certain embodiments, the vaporizer assembly further includes: an outer cylindrical E-liquid medium, an inner cylindrical E-liquid medium, a heating element, and a vaporizer base. The vaporizer base has a cylindrical wall defining a set of vaporizer base E-liquid openings. The outer cylindrical E-liquid medium is positioned outside of the cylindrical wall of the vaporizer base, and the inner cylindrical E-liquid medium is positioned inside of the cylindrical wall of the vaporizer base. The inner cylindrical E-liquid medium receives the E-liquid from the E-liquid storage tank through the outer cylindrical E-liquid medium and the set of vaporizer E-liquid openings. The heating element heats the E-liquid in the inner cylindrical E-liquid medium to generate the E-liquid vapor.

In certain embodiments, the one-way airflow valve includes: a cylindrical one-way airflow valve body, a one-way airflow valve cap, a one-way airflow valve spring, and the one-way airflow blocker. The cylindrical one-way airflow valve body has a top end and a bottom end. The cylindrical one-way airflow valve body defines a set of air outlet openings for discharging airflow through the one-way airflow valve. The one-way airflow valve cap closes the top end of the cylindrical one-way airflow valve body. The bottom end forms the one-way airflow air-intake opening. When the one-way airflow valve is positioned in a vaporizer assembly of the electronic cigarette, the vaporizer assembly forms the airflow path from the one-way airflow air-intake opening at the bottom end of the one-way airflow valve through the set of air outlet openings. The one-way airflow blocker is used to open and close the airflow path. When the one-way airflow blocker is in a first position, the airflow path is closed, and when the one-way airflow blocker is in a second position, the airflow path is open. The one-way airflow valve spring keeps the one-way airflow blocker in the first position. When a user sucks the electronic cigarette, air pressure inside the electronic cigarette is lower than the air pressure of outside air, and this air pressure difference pushes the one-way airflow blocker upwards to the second position to open the airflow path. When the user stops sucking, the one-way airflow blocker returns to the first position under resilient force of the one-way airflow valve spring to close the airflow path.

These and other aspects of the present disclosure will become apparent from the following description of the preferred embodiment taken in conjunction with the following drawings, although variations and modifications therein may be effected without departing from the spirit and scope of the novel concepts of the disclosure.

### BRIEF DESCRIPTION OF THE DRAWINGS

The accompanying drawings illustrate one or more embodiments of the disclosure and, together with the written description, serve to explain the principles of the disclosure. Wherever possible, the same reference numbers are used throughout the drawings to refer to the same or like elements of an embodiment. The drawings do not limit the present disclosure to the specific embodiments disclosed and described herein. The drawings are not necessarily to scale, emphasis instead being placed upon clearly illustrating the principles of the disclosure, and wherein:
FIG. 1 is a cross-sectional view of a one-way airflow valve according to one embodiment of the present disclosure;
FIG. 2 is a cross-sectional view of a one-way airflow valve according to another embodiment of the present disclosure;
FIG. 3 is a cross-sectional view of an electronic cigarette having a one-way airflow valve according to one embodiment of the present disclosure;
FIG. 4 is an exploded perspective view of a vaporizer assembly having the one-way airflow valve according to certain embodiments of the present disclosure;
FIG. 5 is an exploded perspective view of a mouthpiece assembly, E-liquid storage tank and electric power connector of an electronic cigarette according to certain embodiments of the present disclosure;
FIG. 6 is a cross-sectional view of the electronic cigarette having the one-way airflow valve when an airflow path is open according to certain embodiments of the present disclosure;
FIG. 7 is a cross-sectional view of the electronic cigarette having the one-way airflow valve when an airflow path is closed according to certain embodiments of the present disclosure;
FIG. 8 is a flow chart of an exemplary method of using the electronic cigarette having the one-way airflow valve according to certain embodiments of the present disclosure.

### DETAILED DESCRIPTION

The present disclosure will now be described more fully hereinafter with reference to the accompanying drawings, in which exemplary embodiments of the disclosure are shown. This disclosure may, however, be embodied in many different forms and should not be construed as limited to the embodiments set forth herein. Rather, these embodiments are provided so that this disclosure will be thorough and complete, and will fully convey the scope of the disclosure to those skilled in the art. Like reference numerals refer to like elements throughout.

It will be understood that when an element is referred to as being "on" another element, it can be directly on the other element or intervening elements may be present therebetween. In contrast, when an element is referred to as being "directly on" another element, there are no intervening elements present. As used herein, the term "and/or" includes any and all combinations of one or more of the associated listed items.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the present disclosure.

The terminology used herein is for the purpose of describing particular embodiments only and is not intended to be limiting of the disclosure. As used herein, the singular forms "a", "an" and "the" are intended to include the plural forms as well, unless the context clearly indicates otherwise. It will be further understood that the terms "comprises" and/or "comprising," or "includes" and/or "including" or "has" and/or "having" when used herein, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom", "upper" or "top," and "front" or "back" may be used herein to describe one element's relationship to another element as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower", can therefore, encompasses both an orientation of "lower" and "upper," depending of the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

As used herein, "around", "about" or "approximately" shall generally mean within 20 percent, preferably within 10 percent, and more preferably within 5 percent of a given value or range. Numerical quantities given herein are approximates, meaning that the term "around", "about" or "approximately" can be inferred if not expressly stated.

Many specific details are provided in the following descriptions to make the present disclosure be fully understood, but the present disclosure may also be implemented by using other manners different from those described herein, so that the present disclosure is not limited by the specific embodiments disclosed in the following.

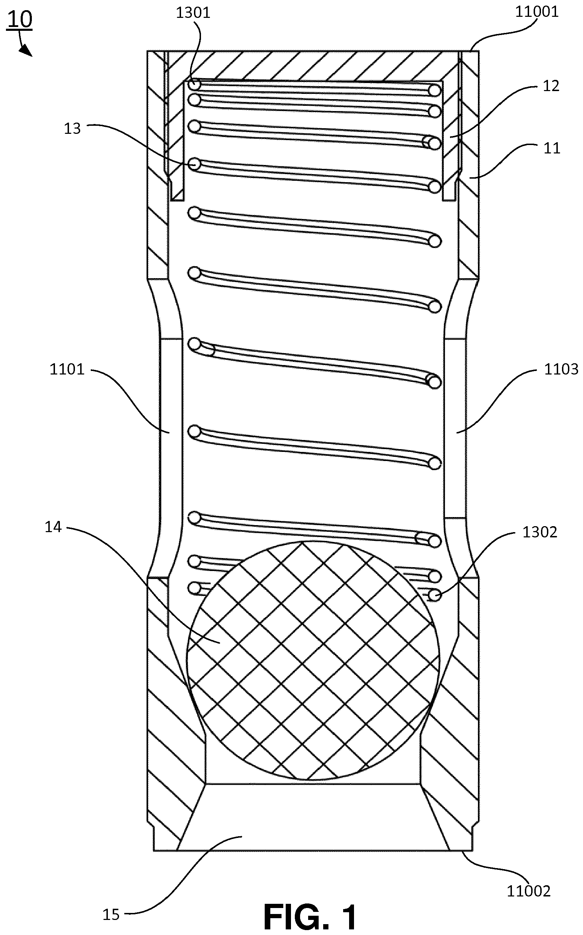

The description will be made as to the embodiments of the present disclosure in conjunction with the accompanying drawings FIGS. 1 through 8.

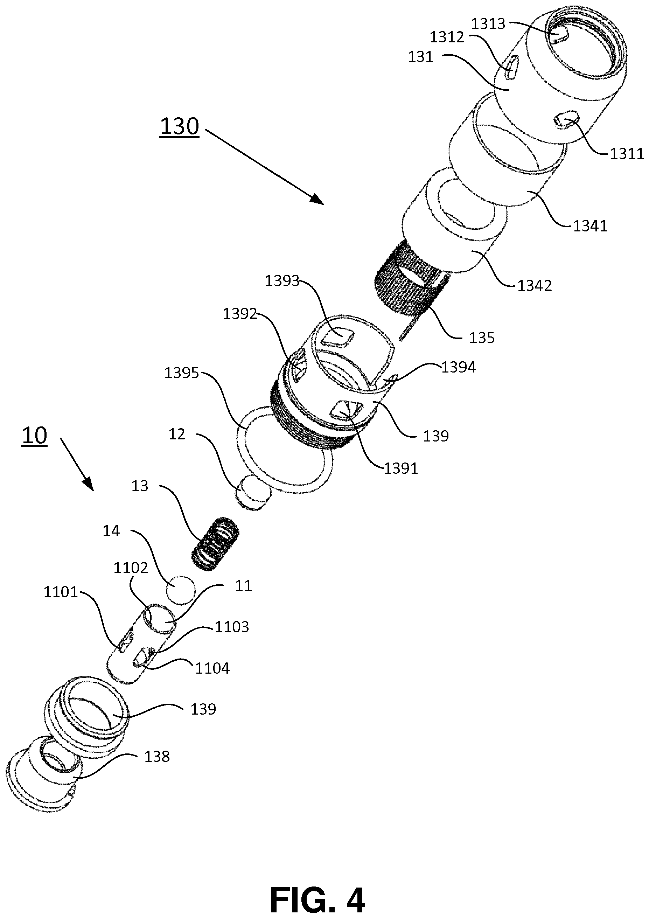

In one aspect, as shown in FIG. 1 through FIG. 7, the present disclosure relates to a one-way airflow valve 10. The one-way airflow valve 10 is used in a vaporizer assembly 130 of an electronic cigarette 100. In certain embodiments, the one-way airflow valve 10 includes: a cylindrical one-way airflow valve body 11, a one-way airflow valve cap 12, a one-way airflow valve spring 13, and a one-way airflow blocker 14. The cylindrical one-way airflow valve body 11 has a top end 11001 and a bottom end 11002. The cylindrical one-way airflow valve body 11 defines a set of air outlet openings for discharging airflow through the one-way airflow valve 10. In certain embodiments, the set of air outlet openings is evenly defined around a side wall of the cylindrical one-way airflow valve body 11. In one embodiment, as shown in FIG. 4, the set of air outlet openings includes: a first air-outlet opening 1101, a second air-outlet opening 1102, a third air-outlet opening 1103, and a fourth air-outlet opening 1104.

In certain embodiments, the one-way airflow valve cap 12 closes the top end 11002 of the cylindrical one-way airflow valve body 11. The bottom end 11002 forms a one-way airflow air-intake opening 15. When the one-way airflow valve 10 is positioned in a vaporizer assembly 130 of an electronic cigarette 100, the vaporizer assembly 130 forms an airflow path from the one-way airflow air-intake opening 15 at the bottom end 11002 of the one-way airflow valve 10 through the set of air outlet openings. The one-way airflow blocker 14 is used to open and close the airflow path.

In certain embodiments, the one-way airflow valve spring 13 includes a first end 1301 and a second end 1302. The first end 1301 is attached to the one-way airflow valve cap 12, and the second end 1302 is attached to the one-way airflow blocker 14. Resilient force of the one-way airflow valve spring 13 keeps the one-way airflow blocker 14 in the first position to close the airflow path. The resilient force of the one-way airflow valve spring 13 is selected to facilitate opening and closing of the airflow path. The resilient force of the one-way airflow valve spring 13 can't be too strong because a too tight one-way airflow valve spring 13 will prevent the airflow path from opening easily. The resilient force of the one-way airflow valve spring 13 can't be too weak either because a too loose one-way airflow valve spring 13 will prevent the airflow path from closing completely.

In certain embodiments, the resilient force of the one-way airflow valve spring 13 is selected so that the user can adjust amount of E-liquid vapor automatically. When the user sucks the mouthpiece 111 harder, the amount of air flowing through the airflow path increases, hence, the user receives more E-liquid vapor at the mouthpiece 111. When the user sucks the mouthpiece 111 lighter, the amount of air flowing through the airflow path decreases, hence, the user receives less E-liquid vapor at the mouthpiece 111. Therefore, the user adjust the amount of E-liquid vapor automatically without manual adjustment of the air intake. In certain embodiments, the one-way airflow blocker 14 is made of: metal, plastic, silicon rubber or wood.

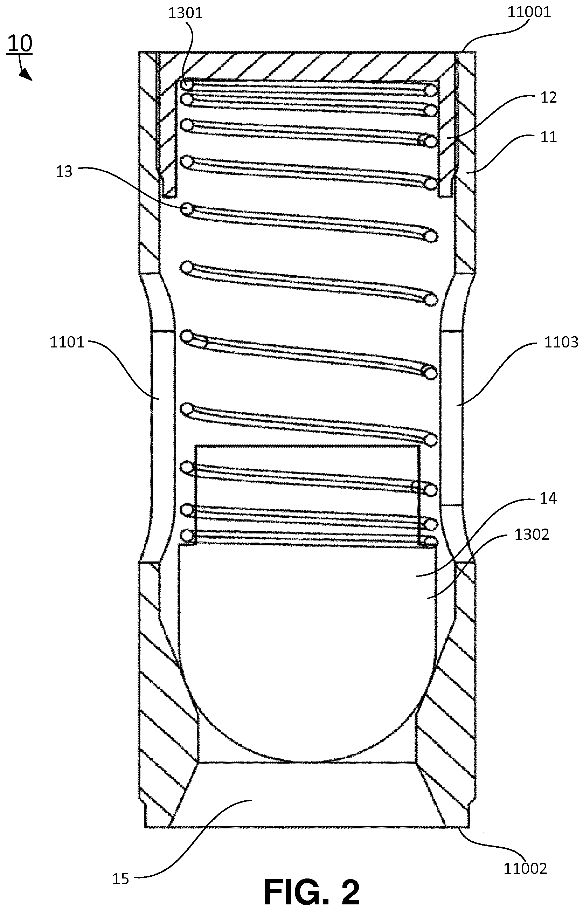

When the one-way airflow blocker 14 is in a first position, the airflow path is closed, and when the one-way airflow blocker 14 is in a second position, the airflow path is open. The one-way airflow valve spring 13 keeps the one-way airflow blocker 14 in the first position. In one embodiment, as shown in FIG. 1, the one-way airflow blocker 14 is a ball to block the airflow path. In another embodiment, as shown in FIG. 2, the one-way airflow blocker 14 is a half ball at the bottom to block the airflow path, and a cylindrical shape on the top to securely attach the one-way airflow valve spring 13. The bottom portion of the cylindrical one-way airflow valve body 11 is shaped to fit the one-way airflow blocker 14 and to completely block the airflow path when the one-way airflow blocker 14 is in the first position.

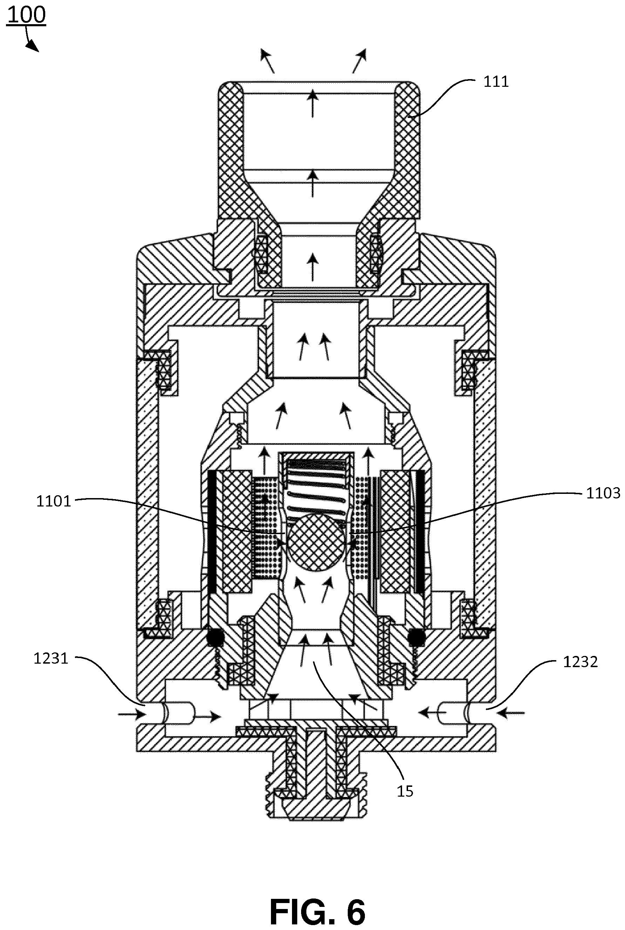

Referring now to FIG. 6, a cross-sectional view of the electronic cigarette having the one-way airflow valve when an airflow path is open is shown according to certain embodiments of the present disclosure. When a user sucks the electronic cigarette 100 through a mouthpiece 111, air pressure inside the electronic cigarette 100 is reduced. The reduction of air pressure creates an air pressure difference and the air pressure difference pushes the one-way airflow blocker 14 of the one-way airflow valve 10 upwards to the second position and opens the airflow path. The outside air enters the electronic cigarette 100 through a first air-intake opening 1231 and a second air-intake opening 1232 of an E-liquid storage tank base 123 and the one-way airflow air-intake opening 15. The air passes around the one-way airflow blocker 14 and the set of air outlet openings 1101, 1102, 1103, and 1104 to enter a vaporizer assembly 130 to be used to vaporize E-liquid siphoned in an inner cylindrical E-liquid medium 1342. The vaporized E-liquid goes up through the mouthpiece 111 for the user to enjoy.

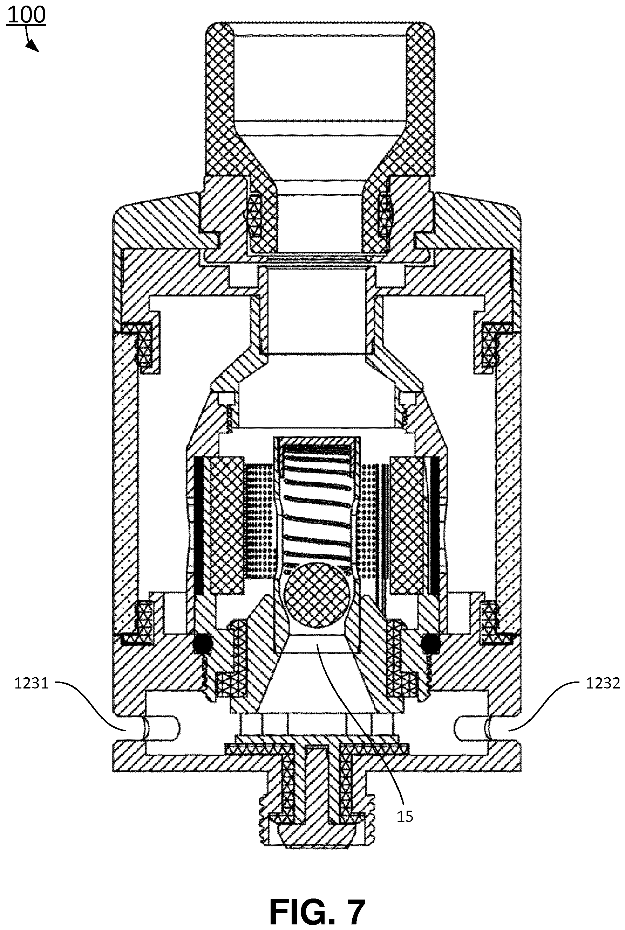

Referring now to FIG. 7, a cross-sectional view of the electronic cigarette having the one-way airflow valve when an airflow path is closed is shown according to certain embodiments of the present disclosure. When the user stops sucking, the one-way airflow blocker 14 returns to the first position under resilient force of the one-way airflow valve spring 13 to close the airflow path. The outside air will not be able to enter the vaporizer assembly 130 through the first air-intake opening 1231 and the second air-intake opening 1232 of the E-liquid storage tank base 123 and the one-way airflow air-intake opening 15.

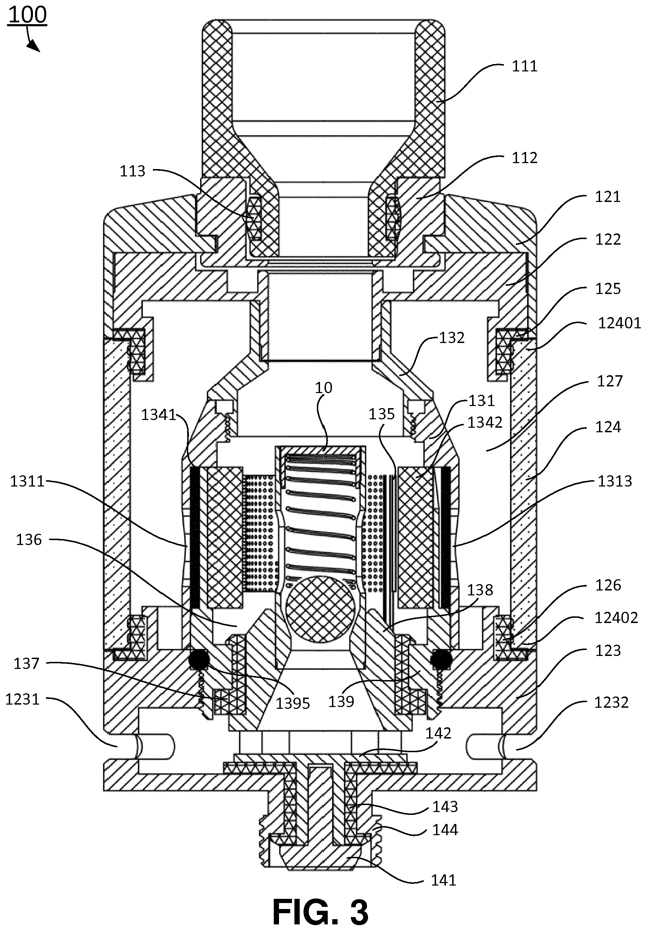

In another aspect, as shown in FIG. 3 through FIG. 7, the present disclosure relates to an electronic cigarette 100. In certain embodiments, the electronic cigarette 100 includes: a mouthpiece assembly 110, an E-liquid storage tank 120, a vaporizer assembly 130, a condensed E-liquid vapor collection slot 136, and an electric power connector 140. The mouthpiece assembly 110 is used for a user to enjoy E-liquid vapor. The E-liquid storage tank 120 stores E-liquid 127 for the electronic cigarette 100. The vaporizer assembly 130 includes a one-way airflow valve 10 for improve the user experience and the condensed E-liquid vapor collection slot 136 to prevent E-liquid leaking and collect condensed E-liquid vapor to be reused. The electric power connector 140 receives electric power from an electric power source and provides the electric power to the vaporizer assembly 130 to vaporize the E-liquid 127.

In certain embodiments, the mouthpiece assembly 110 includes the mouthpiece 111 and a mouthpiece mounting base 112. The mouthpiece 111 is mounted on the mouthpiece mounting base 112. The mouthpiece 111 is sealed by a mouthpiece sealing ring 113 to prevent E-liquid vapor to leak from the mouthpiece assembly 110.

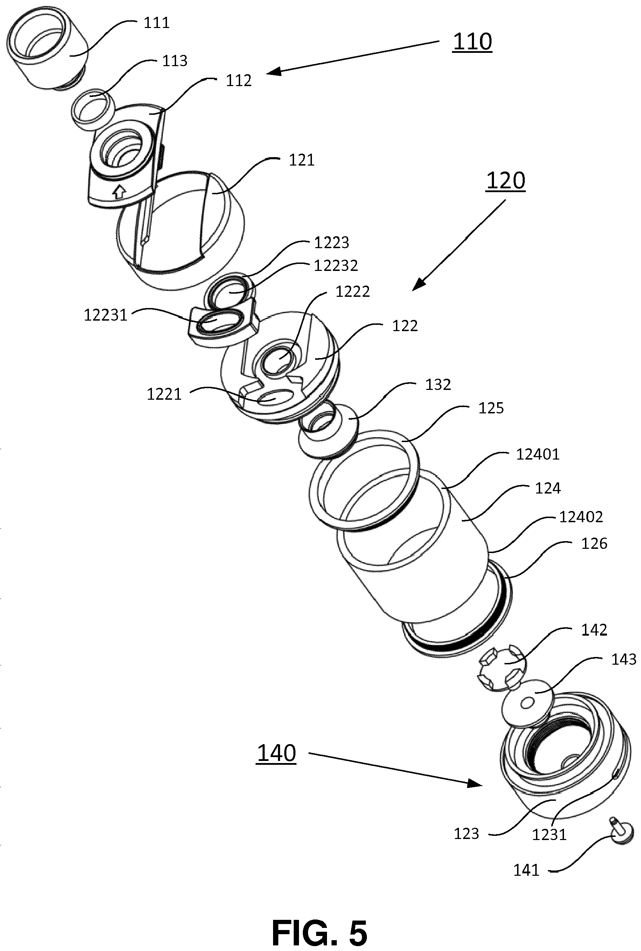

In certain embodiments, as shown in FIG. 3 and FIG. 5, the E-liquid storage tank 120 includes: an E-liquid storage tank top outer cover 121, an E-liquid storage tank top inner cover 122, an E-liquid storage tank external wall 124, an E-liquid storage tank base 123, and a vaporizer assembly body 131. The E-liquid storage tank top outer cover 121 and the E-liquid storage tank top inner cover 122 form a top cover of the E-liquid storage tank 120. The E-liquid storage tank base 123 forms a bottom cover of the E-liquid storage tank 120.

In certain embodiments, the mouthpiece mounting base 112 is slidably attached to the E-liquid storage tank top outer cover 121. The E-liquid storage tank top inner cover 122 defines an E-liquid refill opening 1221 and a center vapor outlet opening 1222. The E-liquid storage tank top inner cover 122 includes a vapor outlet and E-liquid refill cover 1223. The vapor outlet and E-liquid refill cover 1223 defines an E-liquid refill opening 12231 corresponding to the E-liquid refill opening 1221 of the E-liquid storage tank top inner cover 122 and a center vapor outlet opening 12232 corresponding to the center vapor outlet opening 1222 of the E-liquid storage tank top inner cover 122.

In certain embodiments, as shown in FIG. 3 and FIG. 5, the E-liquid storage tank external wall 124 has a top end 12401 and a bottom end 12402. The top end 12401 of the E-liquid storage tank external wall 124 is sealed by an E-liquid storage tank top sealing ring 125, and the bottom end 12402 of the E-liquid storage tank external wall 124 is sealed by an E-liquid storage tank bottom sealing ring 126. In certain embodiments, the E-liquid storage tank external wall 124 may be made of transparent materials such that remaining reserve of the E-liquid 127 in the E-liquid storage tank 120 can be viewed from outside. The transparent materials may include: plastics, acrylics, and glass.

In certain embodiments, the vaporizer assembly body 131 is covered by a vaporizer assembly top vapor outlet 132 on a top end of the vaporizer assembly body 131, and a one-way airflow valve mounting base 138 at a bottom end of the vaporizer assembly body 131. The vaporizer assembly body 131 and the vaporizer assembly top vapor outlet 132 form an E-liquid storage tank 120 internal wall. The vaporizer assembly body 131 defines a set of vaporizer E-liquid openings to allow the E-liquid 127 stored in the E-liquid storage tank 120 to flow through the set of vaporizer E-liquid openings into the vaporizer assembly 130. In one embodiment, as shown in FIG. 5, the set of vaporizer E-liquid openings of the vaporizer assembly body 131 includes: a first vaporizer E-liquid opening 1311, a second vaporizer E-liquid opening 1312, a third vaporizer E-liquid opening 1313, and a fourth vaporizer E-liquid opening 1314. These vaporizer E-liquid openings are evenly defined on a side wall of the vaporizer assembly body 131 to allow the E-liquid 127 to soak into an outer cylindrical E-liquid medium 1341 and an inner cylindrical E-liquid medium 1342.

In certain embodiments, when the E-liquid storage tank 120 needs to be filled with E-liquid, the user slides the mouthpiece mounting base 112 off from the E-liquid storage tank top outer cover 121 to expose the E-liquid refill opening 1221 of the E-liquid storage tank top inner cover 122 and the E-liquid refill opening 12231 of the vapor outlet and E-liquid refill cover 1223. The E-liquid 127 is filled through the E-liquid refill opening 1221 of the E-liquid storage tank top inner cover 122 and the E-liquid refill opening 12231 of the vapor outlet and E-liquid refill cover 1223. The E-liquid 127 in the E-liquid storage tank 120 flows to the vaporizer assembly 130 through the set of vaporizer E-liquid openings.

In certain embodiments, as shown in FIG. 3, and FIG. 4, the vaporizer assembly 130 further includes: the outer cylindrical E-liquid medium 1341, the inner cylindrical E-liquid medium 1342, a heating element 135, a condensed E-liquid vapor collection slot 136 and a vaporizer base 139. In certain embodiments, the outer cylindrical E-liquid medium 1341 and the inner cylindrical E-liquid medium 1342 may include: cotton fibers, polypropylene fibers, terylene fibers, nylon fibers, porous ceramic materials, millipore materials and any combinations of these materials.

The vaporizer base 139 has a cylindrical wall defining a set of vaporizer base E-liquid openings. In certain embodiments, the set of vaporizer base E-liquid openings is evenly defined on the cylindrical wall and includes: a first vaporizer base E-liquid opening 1391, a second vaporizer base E-liquid opening 1392, a third vaporizer base E-liquid opening 1393, and a fourth vaporizer base E-liquid opening 1394. The outer cylindrical E-liquid medium 1341 is positioned outside of the cylindrical wall of the vaporizer base 139, and the inner cylindrical E-liquid medium 1342 is positioned inside of the cylindrical wall of the vaporizer base 139. The inner cylindrical E-liquid medium 1342 receives the E-liquid 127 from the E-liquid storage tank 120 through the outer cylindrical E-liquid medium 1341 and the set of vaporizer E-liquid openings.

In certain embodiments, the heating element 135 heats the E-liquid 127 soaked in the inner cylindrical E-liquid medium 1342 to generate the E-liquid vapor. The heating element 135 is made of one or more resistive materials. When the heating element 135 is powered by the electrical power source through the electric power connector 140, the heating element 135 heats the E-liquid 1027 siphoned through the inner cylindrical E-liquid medium 1342 to generate the E-liquid vapor. The heating element 135 may include: a grid shaped heating element, a mesh shaped heating element, a net shaped heating element, a spiral heating element; and any combination these heating elements. The heating element 135 is surrounded with the E-liquid 127 soaked in the inner cylindrical E-liquid medium 1342 such that the E-liquid 127 in the E-liquid storage tank assembly 102 may be siphoned through the E-liquid 127 soaked in the inner cylindrical E-liquid medium 1342 and heated by the heating element 135.

In certain embodiments, the heating element 135 may be made with one or more of: aluminum (Al), Chromium(Cr), Manganese (Mn), Iron (Fe), Cobalt (Co), Nickel (Ni), Copper (Cu), Zirconium (Zr), Niobium (Nb), Molybdenur (Mo), Rhenium (Re), Silver (Ag), Cadmium(Cd), Tantalum (Ta), Tungsten (W), Iridium (Ir), Platinum (Pt), Gold (Au), and alloys of these materials.

When a conventional electronic cigarette is not in use, the vaporized E-liquid may be condensed and liquified inside its vaporizer, and accumulated condensed E-liquid vapor may cause leaking of condensed and liquified E-liquid vapor out of the vaporizer. In certain embodiments, the condensed E-liquid vapor collection slot 136 is designed to collect the condensed and liquified E-liquid vapor at the bottom of the vaporizer assembly 130 when the electronic cigarette 100 is not in use. The condensed and liquified E-liquid vapor is accumulated in the condensed E-liquid vapor collection slot 136, and is absorbed by the inner cylindrical E-liquid medium 1342. The absorbed condensed E-liquid vapor may be reused next time when the user uses the electronic cigarette 100. Such as reuse prevent waste of condensed E-liquid, and further prevent the leaking of E-liquid from the vaporizer assembly 130.

In certain embodiments, as shown in FIG. 1 through FIG. 4, the one-way airflow valve 10 includes: a cylindrical one-way airflow valve body 11, a one-way airflow valve cap 12, a one-way airflow valve spring 13, and a one-way airflow blocker 14. The cylindrical one-way airflow valve body 11 has a top end 11001 and a bottom end 11002. The cylindrical one-way airflow valve body 11 defines a set of air outlet openings for discharging airflow through the one-way airflow valve 10. In certain embodiments, the set of air outlet openings is evenly defined around a side wall of the cylindrical one-way airflow valve body 11. In one embodiment, as shown in FIG. 4, the set of air outlet openings includes: a first air-outlet opening 1101, a second air-outlet opening 1102, a third air-outlet opening 1103, and a fourth air-outlet opening 1104.

In certain embodiments, the one-way airflow valve cap 12 closes the top end 11002 of the cylindrical one-way airflow valve body 11. The bottom end 11002 forms a one-way airflow air-intake opening 15. When the one-way airflow valve 10 is positioned in a vaporizer assembly 130 of an electronic cigarette 100, the vaporizer assembly 130 forms an airflow path from the one-way airflow air-intake opening 15 at the bottom end 11002 of the one-way airflow valve 10 through the set of air outlet openings. The one-way airflow blocker 14 is used to open and close the airflow path.

When the one-way airflow blocker 14 is in a first position, the airflow path is closed, and when the one-way airflow blocker 14 is in a second position, the airflow path is open. The one-way airflow valve spring 13 keeps the one-way airflow blocker 14 in the first position. In one embodiment, as shown in FIG. 1, the one-way airflow blocker 14 is a ball to block the airflow path. In another embodiment, as shown in FIG. 2, the one-way airflow blocker 14 is a half ball at the bottom to block the airflow path, and a cylindrical shape on the top to securely attach the one-way airflow valve spring 13. The bottom portion of the cylindrical one-way airflow valve body 11 is shaped to fit the one-way airflow blocker 14 and to completely block the airflow path when the one-way airflow blocker 14 is in the first position.

In certain embodiments, the mouthpiece assembly 110 and the vaporizer assembly 130 form an airflow path from a one-way airflow air-intake opening 15 at a bottom end 11002 of the one-way airflow valve 10 through a set of air outlet openings of the one-way airflow valve 10. When the user connects the electric power source to the electric power connector 140, and sucks the electronic cigarette 100 through a mouthpiece 111 of the mouthpiece assembly 110, air pressure inside the electronic cigarette 100 is reduced. The reduction of air pressure creates an air pressure difference and the air pressure difference pushes a one-way airflow blocker 14 of the one-way airflow valve 10 upwards to a second position and opens the airflow path, as shown in FIG. 6. When the user stops sucking, the one-way airflow blocker 14 returns to a first position under resilient force of the one-way airflow valve spring 13 to close the airflow path, as shown in FIG. 7.

Referring now to FIG. 6, a cross-sectional view of the electronic cigarette having the one-way airflow valve when an airflow path is open is shown according to certain embodiments of the present disclosure. When a user sucks the electronic cigarette 100 through a mouthpiece 111, air pressure inside the electronic cigarette 100 is reduced. The reduction of air pressure creates an air pressure difference and the air pressure difference pushes the one-way airflow blocker 14 of the one-way airflow valve 10 upwards to the second position and opens the airflow path. The outside air enters the electronic cigarette 100 through a first air-intake opening 1231 and a second air-intake opening 1232 of an E-liquid storage tank base 123 and the one-way airflow air-intake opening 15. The air passes around the one-way airflow blocker 14 and the set of air outlet openings 1101, 1102, 1103, and 1104 to enter a vaporizer assembly 130 to be used to vaporize E-liquid siphoned in an inner cylindrical E-liquid medium 1342. The vaporized E-liquid goes up through the mouthpiece 111 for the user to enjoy.

Referring now to FIG. 7, a cross-sectional view of the electronic cigarette having the one-way airflow valve when an airflow path is closed is shown according to certain embodiments of the present disclosure. When the user stops sucking, the one-way airflow blocker 14 returns to the first position under resilient force of the one-way airflow valve spring 13 to close the airflow path. The outside air will not be able to enter the vaporizer assembly 130 through the first air-intake opening 1231 and the second air-intake opening 1232 of the E-liquid storage tank base 123 and the one-way airflow air-intake opening 15.

In certain embodiments, the one-way airflow valve spring 13 includes a first end 1301 and a second end 1302. The first end 1301 is attached to the one-way airflow valve cap 12, and the second end 1302 is attached to the one-way airflow blocker 14. Resilient force of the one-way airflow valve spring 13 keeps the one-way airflow blocker 14 in the first position to close the airflow path. The resilient force of the one-way airflow valve spring 13 is selected to facilitate opening and closing of the airflow path. The resilient force of the one-way airflow valve spring 13 can't be too strong because a too tight one-way airflow valve spring 13 will prevent the airflow path from opening easily. The resilient force of the one-way airflow valve spring 13 can't be too weak either because a too loose one-way airflow valve spring 13 will prevent the airflow path from closing completely.

In certain embodiments, the resilient force of the one-way airflow valve spring 13 is selected so that the user can adjust amount of E-liquid vapor automatically. When the user sucks the mouthpiece 111 harder, the amount of air flowing through the airflow path increases, hence, the user receives more E-liquid vapor at the mouthpiece 111. When the user sucks the mouthpiece 111 lighter, the amount of air flowing through the airflow path decreases, hence, the user receives less E-liquid vapor at the mouthpiece 111. Therefore, the user adjust the amount of E-liquid vapor automatically without manual adjustment of the air intake. In certain embodiments, the one-way airflow blocker 14 is made of: metal, plastic, silicon rubber or wood.

In certain embodiments, the electric power connector 140 includes: a positive electric power terminal 141, a negative electric power terminal 142, and an electric power insulator ring 143. The positive electric power terminal 141 connects the heating element 135 to a positive terminal of the electric power source. The negative electric power terminal 142 connects the heating element 135 to a negative terminal of the electric power source. The electric power insulator ring 143 is positioned between the positive electric power terminal 141 and the negative electric power terminal 142 to insulate the positive electric power terminal 141 and the negative electric power terminal 142. In certain embodiments, the vaporizer base 139 forms the negative electric power terminal 142.

A person skilled in the art should appreciate the fact that electric power source is essential to the electronic cigarette 100 and it not shown and discussed in FIGS. 1-7 for brevity. The electric power source includes a positive terminal and a negative terminal. The positive terminal of the electric power source is connected to the positive electric power terminal 141 of the electric power connector 140, and the negative terminal of the electric power source is connected to the negative electric power terminal 142 of the electric power connector 140. In certain embodiments, the electrical power connector 140 may be a T-shaped groove connector, a dovetail shaped slot connector, a magnetic attachment connector, a threaded connector, and a multi-threaded connector.

In certain embodiments, the electrical power source includes: a battery and/or a rechargeable battery. In certain embodiments, the rechargeable battery may include including lead-acid, nickel cadmium (NiCd), nickel metal hydride (NiMH), lithium ion (Li-ion), and lithium ion polymer (Li-ion polymer).

In yet another aspect, the present disclosure relates to a method of using an electronic cigarette 100 having a one-way airflow valve 10. In certain embodiments, the method of using the electronic cigarette 100 includes: sliding off, by a user, a mouthpiece mounting base 112 of the electronic cigarette 100 and filling E-liquid 127 into an E-liquid storage tank 120 through an E-liquid refill opening 1221 on an E-liquid storage tank top inner cover 122, and connecting, by the user, an electric power source to an electric power connector 140 of the electronic cigarette 100. The method of using the electronic cigarette 100 also includes: turning on, by the user, the electric power source to power a vaporizer assembly 130 of the electronic cigarette 100 having the one-way airflow valve 10, sucking, by the user, through a mouthpiece 111 to open an airflow path through the one-way airflow valve 10 and enjoying the vapor generated by the vaporizer assembly 130, and stopping sucking through the mouthpiece 111 to close the airflow path through the one-way airflow valve 10 such that the airflow through the vaporizer assembly 130 stops.

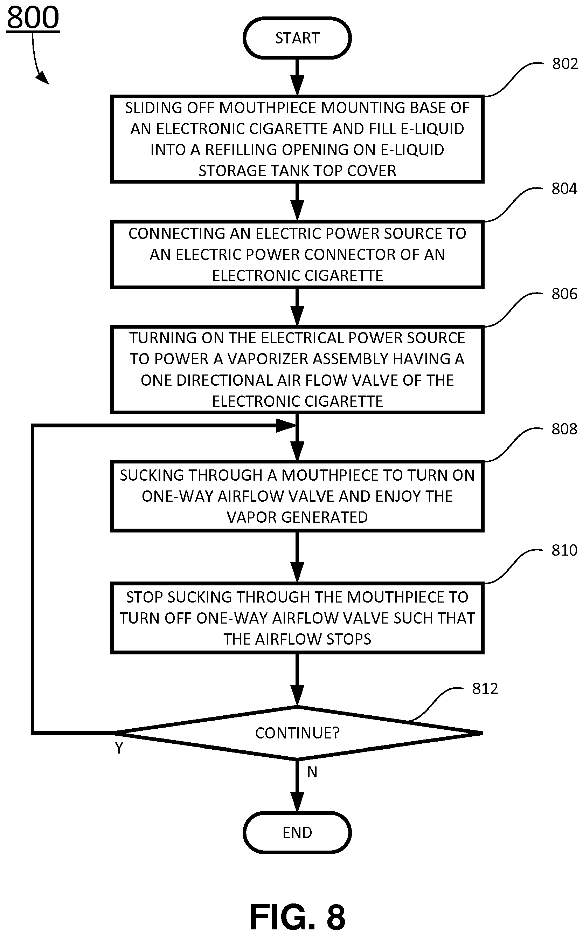

In yet another aspect, the present disclosure relates to method 800 of using an electronic cigarette 100 having a one-way airflow valve 10 as shown in FIG. 8.

At block 802, a user slides a mouthpiece mounting base 112 of the electronic cigarette 100 off an E-liquid storage tank top outer cover 121 to expose an E-liquid refill opening 1221 of an E-liquid storage tank top inner cover 122 and an E-liquid refill opening 12231 of a vapor outlet and E-liquid refill cover 1223. The user fills E-liquid into an E-liquid storage tank 120 through the E-liquid refill opening 1221 of the E-liquid storage tank top inner cover 122 and the E-liquid refill opening 12231 of the vapor outlet and E-liquid refill cover 1223.

At block 804, the user connects the electrical power source to an electric power connector 140 of the electronic cigarette 100. The electrical power connector 140 may be a T-shaped groove connector, a dovetail shaped slot connector, a magnetic attachment connector, a threaded connector, and a multi-threaded connector. In one embodiment shown in FIG. 3, the electric power connector 140 is a threaded connector.

At block 806, the user turns on the electrical power source to power a vaporizer assembly 130 having the one-way airflow valve 10.

At block 808, the user sucks the electronic cigarette 100 through a mouthpiece 111, air pressure inside the electronic cigarette 100 is reduced. The reduction of air pressure creates an air pressure difference and the air pressure difference pushes a one-way airflow blocker 14 of the one-way airflow valve 10 upwards to a second position and opens an airflow path. The outside air enters the electronic cigarette 100 through a first air-intake opening 1231 and a second air-intake opening 1232 of an E-liquid storage tank base 123 and a one-way airflow air-intake opening 15. The air passes around the one-way airflow blocker 14 and a set of air outlet openings 1101, 1102, 1103, and 1104 to enter the vaporizer assembly 130 to be used to vaporize E-liquid siphoned in an inner cylindrical E-liquid medium 1342. The vaporized E-liquid goes up through the mouthpiece 111 for the user to enjoy.

At block 810, when the user stops sucking the electronic cigarette 100 through the mouthpiece 111, the one-way airflow blocker 14 returns to a first position under resilient force of the one-way airflow valve spring 13 to close the airflow path. The outside air will not be able to enter the vaporizer assembly 130 through the first air-intake opening 1231 and the second air-intake opening 1232 of the E-liquid storage tank base 123 and the one-way airflow air-intake opening 15.

At query block 812, the user decides whether the user wants to continue using the electronic cigarette 100. When the user decides to continue using the electronic cigarette 100, the method proceeds to block 808 to continue. Otherwise, the method proceeds to end the use of the electronic cigarette 100.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its spirit and scope. Accordingly, the scope of the present disclosure is defined by the appended claims rather than the foregoing description and the exemplary embodiments described therein.

## Claims

1. A one-way airflow valve, comprising:
a cylindrical one-way airflow valve body having a top end, a bottom end, wherein the cylindrical one-way airflow valve body defines a plurality of air outlet openings for discharging airflow through the one-way airflow valve, wherein the plurality of air outlet openings comprises: a first air-outlet opening, a second air-outlet opening, a third air-outlet opening, and a fourth air-outlet opening, and the plurality of air outlet openings is evenly defined around a side wall of the cylindrical one-way airflow valve body;
a one-way airflow valve cap for closing the top end of the cylindrical one-way airflow valve body;
a one-way airflow blocker for opening and closing one-way airflow, wherein when the one-way airflow valve is positioned in a vaporizer assembly of an electronic cigarette, the vaporizer forms an airflow path from an one-way airflow air-intake opening at the bottom end of the one-way airflow valve through the plurality of air outlet openings, when the one-way airflow blocker is in a first position, the airflow path is closed, and when the one-way airflow blocker is in a second position, the airflow path is open, wherein the one-way airflow blocker comprises: metal, plastic, silicon rubber and wood; and
a one-way airflow valve spring for keeping the one-way airflow blocker in the first position, wherein the one-way airflow valve spring comprises a first end attached to the one-way airflow valve cap, and a second end attached to the one-way airflow blocker, wherein the resilient force of the one-way airflow valve spring keeps the one-way airflow blocker in the first position to close the airflow path, and the resilient force of the one-way airflow valve spring is selected to facilitate opening and closing of the airflow path and allow the user to adjust the amount of airflow through the one-way airflow valve and amount of E-liquid vaper according to suction force applied in real-time automatically,
wherein when a user sucks the electronic cigarette through a mouthpiece, air pressure inside the electronic cigarette is reduced, the reduction of air pressure creates an air pressure difference, and the air pressure difference pushes the one-way airflow blocker of the one-way airflow valve upwards to the second position and opens the airflow path, and when the user stops sucking, the one-way airflow blocker returns to the first position under resilient force of the one-way airflow valve spring to close the airflow path.

2. An electronic cigarette, comprising:
a mouthpiece assembly for a user to enjoy E-liquid vapor;
an E-liquid storage tank for storing E-liquid;
a vaporizer assembly having a one-way airflow valve;
an electric power connector to receive electric power from an electric power source and to provide the electric power to the vaporizer assembly; and
a condensed E-liquid vapor collection slot for collecting condensed E-liquid vapor to be reused,
wherein the mouthpiece assembly and the vaporizer assembly form an airflow path from an one-way airflow air-intake opening at a bottom end of the one-way airflow valve through a plurality of air outlet openings of the one-way airflow valve, when the user connects the electric power source to the electric power connector, and sucks the electronic cigarette through a mouthpiece, air pressure inside the electronic cigarette is reduced, the reduction of air pressure creates an air pressure difference, and the air pressure difference pushes a one-way airflow blocker of the one-way airflow valve upwards to a second position and opens the airflow path, and when the user stops sucking, the one-way airflow blocker returns to a first position under resilient force of the one-way airflow valve spring to close the airflow path.

3. The electronic cigarette of claim 2, wherein the mouthpiece assembly comprises a mouthpiece positioned on a mouthpiece mounting base, and sealed by a mouthpiece sealing ring.

4. The electronic cigarette of claim 3, wherein the E-liquid storage tank comprises:
an E-liquid storage tank top outer cover, wherein the mouthpiece mounting base is slidably attached to the E-liquid storage tank top outer cover;
an E-liquid storage tank top inner cover, wherein the E-liquid storage tank top inner cover defines an E-liquid refill opening, and a center vapor outlet opening, and comprises a vapor outlet and E-liquid refill cover defining an E-liquid refill opening corresponding to the E-liquid refill opening of the E-liquid storage tank top inner cover and a center vapor outlet opening corresponding to the center vapor outlet opening of the E-liquid storage tank top inner cover;
an E-liquid storage tank external wall, sealed by an E-liquid storage tank top sealing ring on a top end of the E-liquid storage tank external wall, and an E-liquid storage tank bottom sealing ring on a bottom end of the E-liquid storage tank external wall;
an E-liquid storage tank base forming a bottom cover of the E-liquid storage tank; and
a vaporizer assembly body covered by a vaporizer assembly top vapor outlet on a top end of the vaporizer assembly body, and a one-way airflow valve mounting base at a bottom end of the vaporizer assembly body, wherein the vaporizer assembly body and the vaporizer assembly top vapor outlet form an E-liquid storage tank internal wall, and the vaporizer assembly body defines a plurality of vaporizer E-liquid openings to allow the E-liquid stored in the E-liquid storage tank to flow through the plurality of vaporizer E-liquid openings into the vaporizer assembly,
wherein the E-liquid is filled through the E-liquid refill opening of the E-liquid storage tank top inner cover and the E-liquid refill opening of a vapor outlet and E-liquid refill cover and flows to the vaporizer assembly through the plurality of vaporizer E-liquid openings.

5. The electronic cigarette of claim 4, wherein the vaporizer assembly further comprises:
an outer cylindrical E-liquid medium and an inner cylindrical E-liquid medium for receiving the E-liquid from the E-liquid storage tank;
a heating element positioned inside of the inner cylindrical E-liquid medium for heating the E-liquid received in the outer cylindrical E-liquid medium and the inner cylindrical E-liquid medium to generate the E-liquid vapor; and
a vaporizer base having a cylindrical wall defining a plurality of vaporizer base E-liquid openings,
wherein the outer cylindrical E-liquid medium is positioned outside of the cylindrical wall, and the inner cylindrical E-liquid medium is positioned inside of the cylindrical wall of the vaporizer base, the inner cylindrical E-liquid medium receives the E-liquid from the E-liquid storage tank through the outer cylindrical E-liquid medium and the set of vaporizer E-liquid openings, and the heating element heats the E-liquid in the inner cylindrical E-liquid medium to generate the E-liquid vapor.

6. The electronic cigarette of claim 5, wherein the one-way airflow valve comprises:
a cylindrical one-way airflow valve body having a top end and a bottom end, wherein the cylindrical one-way airflow valve body defines the plurality of air outlet openings for discharging airflow through the one-way airflow valve;
a one-way airflow valve cap for closing the top end of the cylindrical one-way airflow valve body;
the one-way airflow blocker, when the one-way airflow blocker is in a first position, an airflow is closed, and when the one-way airflow blocker is in a second position, the airflow is open for opening and closing one-way airflow, wherein the one-way airflow blocker comprises: metal, plastic, silicon rubber and wood; and
a one-way airflow valve spring for keeping the one-way airflow blocker in the first position,
wherein the one-way airflow valve is positioned in the vaporizer assembly, the vaporizer assembly forms the airflow path from the one-way airflow air-intake opening at the bottom end of the one-way airflow valve through the plurality of air outlet openings, when the user sucks the electronic cigarette through a mouthpiece, air pressure inside the electronic cigarette is reduced, the reduction of air pressure creates an air pressure difference, and the air pressure difference pushes the one-way airflow blocker of the one-way airflow valve upwards to the second position and opens the airflow path, and when the user stops sucking, the one-way airflow blocker returns to the first position under resilient force of the one-way airflow valve spring to close the airflow path.

7. The electronic cigarette of claim 6, wherein the plurality of air outlet openings is evenly defined around a side wall of the cylindrical one-way airflow valve body, and comprises: a first air-outlet opening, a second air-outlet opening, a third air-outlet opening, and a fourth air-outlet opening.

8. The electronic cigarette of claim 6, wherein the one-way airflow valve spring comprises a first end attached to the one-way airflow valve cap, and a second end attached to the one-way airflow blocker, wherein the resilient force of the one-way airflow valve spring keeps the one-way airflow blocker in the first position to close the airflow path.

9. The electronic cigarette of claim 6, wherein the resilient force of the one-way airflow valve spring is selected to facilitate opening and closing of the airflow path and allow the user to adjust the amount of airflow through the one-way airflow valve and amount of E-liquid vaper according to suction force applied in real-time automatically.

10. The electronic cigarette of claim 6, wherein the electric power connector comprises:
a positive electric power terminal for connecting the heating element to a positive terminal of the electric power source;
a negative electric power terminal for connecting the heating element to a negative terminal of the electric power source, wherein the vaporizer base forms the negative electric power terminal; and
an electric power insulator ring, positioned between the positive electric power terminal and the negative electric power terminal to insulate the positive electric power terminal and the negative electric power terminal.

11. The electronic cigarette of claim 10, wherein the heating element comprises one or more resistive materials, when the heating element is powered by the electric power source through the positive electric power terminal and the negative electric power terminal, the heating element heats the E-liquid siphoned through the outer cylindrical E-liquid medium and the inner cylindrical E-liquid medium to generate the E-liquid vapor.

12. A method of using an electronic cigarette having a one-way airflow valve, comprising:
sliding off, by a user, a mouthpiece mounting base of the electronic cigarette from an E-liquid storage tank top outer cover to expose an E-liquid refill opening of an E-liquid storage tank top inner cover and an E-liquid refill opening of a vapor outlet and E-liquid refill cover, and filling E-liquid into an E-liquid storage tank through the E-liquid refill opening on the E-liquid storage tank top inner cover and the E-liquid refill opening of the vapor outlet and E-liquid refill cover;
connecting, by the user, an electric power source to an electric power connector of the electronic cigarette;
turning on, by the user, the electric power source to power a vaporizer assembly of the electronic cigarette having the one-way airflow valve;
sucking, by the user, through a mouthpiece to open an airflow path through the one-way airflow valve, and enjoying the vapor generated by the vaporizer assembly; and
stopping sucking through the mouthpiece to close the airflow path and the airflow through the vaporizer assembly stops.

13. The method of claim 12, wherein the electronic cigarette comprises:
a mouthpiece assembly for the user to enjoy E-liquid vapor;
the E-liquid storage tank for storing E-liquid;
a vaporizer assembly having a one-way airflow valve; and
the electric power connector to receive electric power from an electric power source and to provide the electric power to the vaporizer assembly,
wherein the mouthpiece assembly and the vaporizer assembly form the airflow path from an one-way airflow air-intake opening at a bottom end of the one-way airflow valve through a plurality of air outlet openings of the one-way airflow valve, when the user connects the electric power source to the electric power connector, and sucks the electronic cigarette through the mouthpiece assembly, air pressure inside the electronic cigarette is reduced, the reduction of air pressure creates an air pressure difference, and the air pressure difference pushes a one-way airflow blocker upwards to a second position and opens the airflow path, and when the user stops sucking, the one-way airflow blocker returns to a first position under resilient force of the one-way airflow valve spring to close the airflow path.

14. The method of claim 13, wherein the vaporizer assembly further comprises:
an outer cylindrical E-liquid medium and an inner cylindrical E-liquid medium for receiving the E-liquid from the E-liquid storage tank;
a heating element positioned inside of the inner cylindrical E-liquid medium for heating the E-liquid received in the outer cylindrical E-liquid medium and the inner cylindrical E-liquid medium to generate the E-liquid vapor; and
a vaporizer base having a cylindrical wall defining a plurality of vaporizer base E-liquid openings,
wherein the outer cylindrical E-liquid medium is positioned outside of the cylindrical wall, and the inner cylindrical E-liquid medium is positioned inside of the cylindrical wall of the vaporizer base, the inner cylindrical E-liquid medium receives the E-liquid from the E-liquid storage tank through the outer cylindrical E-liquid medium and the set of vaporizer E-liquid openings, and the heating element heats the E-liquid in the inner cylindrical E-liquid medium to generate the E-liquid vapor.

15. The method of claim 13, wherein the one-way airflow valve comprises:
a cylindrical one-way airflow valve body having a top end and a bottom end, wherein the cylindrical one-way airflow valve body defines the plurality of air outlet openings for discharging airflow through the one-way airflow valve;
a one-way airflow valve cap for closing the top end of the cylindrical one-way airflow valve body;
the one-way airflow blocker, when the one-way airflow blocker is in a first position, an airflow is closed, and when the one-way airflow blocker is in a second position, the airflow is open for opening and closing one-way airflow;
a one-way airflow valve spring for keeping the one-way airflow blocker in the first position,
wherein the one-way airflow valve is positioned in the vaporizer assembly, the vaporizer assembly forms the airflow path from the one-way airflow air-intake opening at the bottom end of the one-way airflow valve through the plurality of air outlet openings, when the user sucks the electronic cigarette 100 through a mouthpiece 111, air pressure inside the electronic cigarette 100 is reduced, the reduction of air pressure creates an air pressure difference, and the air pressure difference pushes a one-way airflow blocker of the one-way airflow valve upwards to the second position and opens the airflow path, and when the user stops sucking, the one-way airflow blocker 14 returns to the first position under resilient force of the one-way airflow valve spring 13 to close the airflow path.
